# EUROPEAN PATENT APPLICATION

(11) **EP 1 464 889 A2**
(43) Date of publication of application: **06.10.2004**
(21) Application number: 04101251.9
(22) Date of filing: 26.03.2004
(51) Int. Cl.: F21S 10/00, F21V 33/00

(54) **Device to illuminate water jets in a sanitary appliance such as a bathtub, a shower cubicle or suchlike**

(30) Priority: 27.03.2003 IT UD20030071
(71) Applicant: Calipso Srl, 33080 Porcia (PN) (IT)
(72) Inventor: Saccon, Luciano, 33074, Fontanafredda (PN) (IT)
(74) Representative: Petraz, Gilberto

(57) **Abstract**

Device (10) to diffuse light in a sanitary appliance (11) such as a bathtub, a shower cubicle or suchlike, comprising a source of light (13). The source of light (13) is installed near a source of water (12) in order to colour the water delivered, and is connected to its own electronic pilot unit (15) in order to regulate individually at least one parameter of light emission, such as the light intensity, wavelength, or switch-on frequency.

## Description

### FIELD OF THE INVENTION

The present invention concerns a device to diffuse light in a sanitary appliance such as a bathtub, a shower cubicle or suchlike. The light is diffused both for purely aesthetic reasons and also for the application of chromatic or phototherapeutic techniques, and also for functional reasons, for example to indicate the approximate temperature of the water. The present invention is applied preferentially, but not exclusively, in so-called hydro-massage bathtubs and shower cubicles, in order to illuminate from behind, in either monochrome or polychrome manner, the mouths and nozzles through which the water and/or air is delivered under pressure.

### BACKGROUND OF THE INVENTION

Sanitary appliances are known, such as bathtubs and shower cubicles for hydro-massage provided with one or more sources of light emitting monochrome or polychrome lights, in order to apply chromotherapy techniques, or simply for aesthetic reasons.

Such sources of light must obviously be well insulated and water tight in order to avoid every minimal contact with the water, and normally consist of colored halogen lamps.

Such halogen lamps have a very high energy consumption, however, they get very hot and last a limited time, thus entailing for the user high maintenance and upkeep expenses, also because it is difficult to access them from the outside.

To limit the emission of heat by halogen lamps, it is known to use optical fibers, by means of which it is possible to distribute the light in multiple points without any emission of heat. Optical fibers, however, are very expensive both to produce and to manage and they are very expensive to use.

It is also known to use a plurality of lamps, or radiating bodies, each of which comprises a corresponding plurality of semi-conductor diodes, commonly known by their acronym "LED" (Light Emitting Diode), which have lower energy consumption and do not emit high quantities of heat.

Each of said lamps, or radiating bodies, is managed by a single electronic pilot unit, common for all the LEDs, and this makes it impossible to deliver a high current for each LED, thus limiting the luminosity of the lamps or radiating bodies. Moreover, since they have an electronic unit in common, it is not possible to manage every LED individually, which precludes the possibility of creating plays of light or particular chromatic effects in each individual lamp or radiating body. Furthermore, if a single LED is broken, it prejudices the functioning and entails the need for intervention on the whole lamp or radiating body.

Another disadvantage of this known solution is that, since the LEDs are connected to a single electronic pilot unit, they cannot be positioned and distributed at particular points of the sanitary appliance, such as for example behind the water delivery nozzles, in order to create a colored effect in the water delivered.

EP-A-1.038.484, on which the preamble of the independent claim 1 is based, discloses a bathtub having a wall on which a plurality of LED's are mounted.

One purpose of the present invention is to achieve a device to diffuse light in a sanitary appliance such as a bathtub, a shower cubicle or suchlike, which has limited energy consumption and management costs, which does not emit high quantities of heat, and in which every individual source of light can be managed individually, or possibly in differentiated manner, in order to permit the creation of plays of light and particular chromatic effects.

More particularly, a purpose of the invention is to create chromatic and lighting plays by providing a plurality of sources of colored water, each of which having the same or a different color of one adjacent water source, these water sources being distributed on the walls of the bathtub or of the shower cubicle.

Another purpose of the present invention is to achieve a device that allows to position and distribute the sources of light easily, substantially at any point whatsoever of the sanitary appliance, and in particular in correspondence of a water source, such as a mouth or a nozzle.

The Applicant has devised, tested and embodied the present invention to overcome the shortcomings of the state of the art and to obtain other purposes and advantages.

### SUMMARY OF THE INVENTION

The present invention is set forth and characterized in the main claim, while the dependent claims describe other characteristics of the present invention or variants to the main idea of the invention.

In accordance with the aforesaid purposes a device according to the present invention to diffuse light in a sanitary appliance such as a bathtub, a shower cubicle or suchlike, comprises at least a source of light, such as a semi-conductor diode, or LED, connected to its own electronic pilot unit, which allows to regulate individually at least one parameter of light emission, such as for example the intensity of the light, the wavelength, the switch-on frequency or otherwise.

To be more exact, the present invention comprises a plurality of sources of light, each one connected to a relative electronic pilot unit.

According to a feature of the invention, each source of light is installed near a delivery nozzle, mouth or other kind of water source. In this way, by switching on the relative source of light, also in differentiated and sequential manner, a desired effect is created to color or illuminate the jets of water delivered from a plurality of points distributed along the wall of the bathtub or of the shower cubicle.

A first advantage of the device according to the present invention is that it allows to manage individually, and possibly in differentiated manner, a plurality of LED-type sources of light coupled with respective sources of water, on the one hand limiting energy consumption and on the other hand allowing to manage the emission parameters of every individual LED in order to create desired plays of light or particular chromatic effects by the jets of water delivered.

Another advantage is that every LED, and the relative electronic unit, can be arranged easily and quickly substantially at any point whatsoever of the sanitary appliance, in particular behind the water delivery nozzles.

Another advantage is that every individual LED can be replaced and maintained without intervening on the others; if one LED is broken, it does not prejudice the functioning of the others.

The intensity of current supplied to each LED can be high if compared to the case of a lamp or radiating body, with the advantage of a greater luminosity emitted.

A further advantage of the present invention is that the limited bulk of each individual LED and its autonomy and independence with respect to the other sources of light allows the direct coupling thereof, for example using constraining systems with a trigger, magnet, bayonet or otherwise, to a relative water delivery mouth or nozzle. On the one hand this prevents the LEDs from coming into contact with the water, and on the other hand it allows to obtain a particular effect of illumination from the rear for every single nozzle.

### BRIEF DESCRIPTION OF THE DRAWINGS

These and other characteristics of the present invention will become apparent from the following description of a preferential form of embodiment, given as a non-restrictive example, with reference to the attached drawings wherein:
- fig. 1 is a three-dimensional view of a hydro-massage shower to which a device to diffuse light according to the present invention is applied;
- fig. 2 is a schematic view of a lateral section of a detail of a water delivery nozzle to which the device in fig. 1 is applied.

### DETAILED DESCRIPTION OF A PREFERENTIAL FORM OF EMBODIMENT

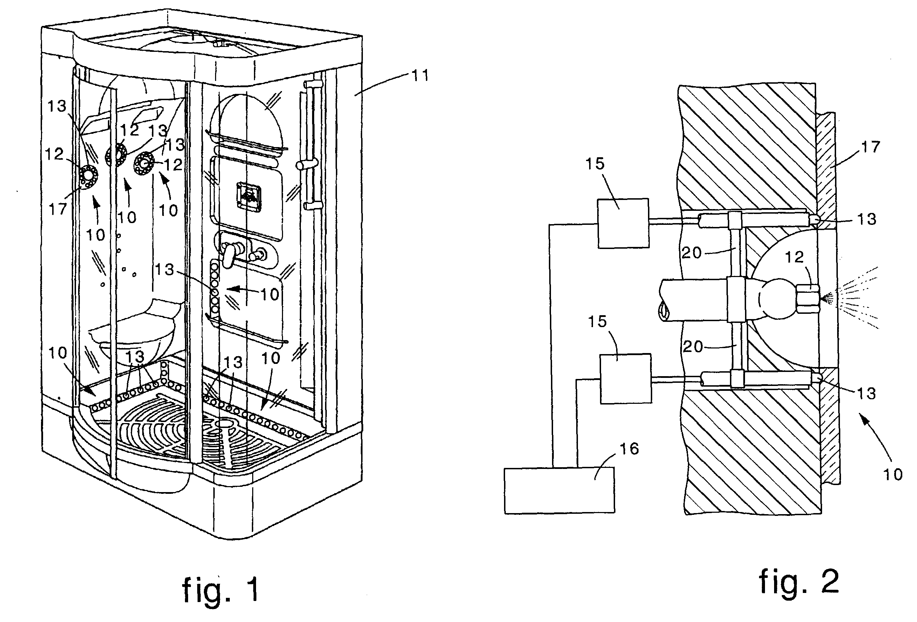

With reference to fig. 1, a device 10 according to the present invention is applied, in this case, to a hydro-massage shower cubicle 11, of the type provided with a plurality of adjustable nozzles 12 that deliver water, or in the case of bathtubs a mix of air and water, under pressure.

The shower cubicle 11 is of a substantially known type, and the details of its construction are not part of the present invention and therefore will not be described in detail here.

The device 10 according to the present invention comprises a plurality of white, monochrome or polychrome LEDs 13, arranged according to a design, in various zones inside the shower cubicle, such as for example along the perimeter of the base plate of the shower, near the technical compartments, on the roof, but in particular near the nozzles 12.

Each LED 13 is connected to its own electronic pilot unit 15 (fig. 2), by means of which it is possible to regulate the intensity of the feed current in order to vary the degree of luminescence of the respective LED 13, or other functioning parameters, for example the frequency and/or times the LED is switched on, the wavelength of the light emitted or otherwise.

The electronic pilot units 15 are connected to a single command and control unit 16, selectively programmable by a user, so as to be able to personalize the program and switching on of the various LEDs 13, for example by associating the switching on to the water delivery mode, or the color and intensity of the lights emitted by the individual LEDs 13.

The device 10 according to the present invention is installed, as shown schematically in fig. 2, near a delivery nozzle 12 around which a disk 17 of transparent material is provided, illuminated from behind by a plurality of LEDs 13. In this way, by switching on the LEDs 13, also in differentiated and sequential manner, a desired effect is created to color or illuminate the jets of water delivered. Each jet of water can assume the same or a different color with respect to the adjacent jet of water, and the color of each jet of water can change, thus allowing to create amazing plays of light and particular chromatic effects.

By using several colored LEDs 13 combined with each other according to the RGB technique, it is possible to obtain the desired colored emissions using a reduced number of LEDs.

In the embodiment shown in fig. 2, the LEDs 13 are directly associated with the nozzle 12 by means of a trigger-type coupling element 20, and their luminous element is located in correspondence with the transparent disk 17. In this way, the nozzle 12 and the relative LED 13 substantially constitute a single body, which considerably facilitates the positioning and assembly operations. Moreover, the fixed position of the LEDs 13 with respect to the nozzle 12 prevents possible contacts with the water and ensures an effective rear illumination effect.

Since the LEDs 13 are managed individually by the relative electronic pilot units 15, they can have different luminous intensity and/or colors, and in the event of a malfunction, the single malfunctioning LED 13 is replaced, without entailing any problem for the remaining LEDs 13 installed in the shower cubicle 11.

Moreover, the device 10 is of extremely limited size, so that the LEDs 13 can be arranged substantially in any zone whatsoever of the shower cubicle 11 without problems of space connected to the bulk of the components.

It is clear, however, that modifications and/or additions of parts may be made to the device 10 as described heretofore, without departing from the field and scope of the present invention.

For example, according to a variant, the disk 17 can provide a prism or a reflecting wall in order to increase the luminous and/or chromatic effect achieved by the LEDs 13.

It is also clear that, although the present invention has been described with reference to specific examples, a skilled person in the art shall certainly be able to achieve many other equivalent forms of device to diffuse light in a sanitary appliance, all of which shall come within the field and scope of the invention.

For example, even though the present description refers to an application in a shower cubicle 11, it can be transferred to a bathtub, a swimming pool, to a sauna cabin or other similar or comparable sanitary appliance.

## Claims

1. Device to diffuse light in a sanitary appliance (11) such as a bathtub, a shower cubicle or suchlike, said sanitary appliance (11) comprising at least a wall on which at least a source of water (12) is provided, the device comprising at least a source of light (13), **characterized in that** said source of light (13) is installed near said source of water (12) in order to color the relative jet of water delivered, and is connected to its own electronic pilot unit (15) able to regulate individually at least one parameter of light emission.

2. Device as in claim 1, **characterized in that** it comprises a plurality of sources of light (13) each of which is installed near a relative source of water (12), each one of the sources of light (13) being able to be connected to a relative electronic pilot unit (15).

3. Device as in claim 1, **characterized in that** said source of light comprises a semi-conductor diode, or LED (13).

4. Device as in claim 1, **characterized in that** said parameter of emission is one of the following: the intensity of the light, the wavelength, or the frequency and/or temperature of emission.

5. Device as in claim 1, **characterized in that** it comprises a programmable command and control unit (16) able to selectively activate every electronic pilot unit (15).

6. Device as in claim 1, **characterized in that** said source of light (13) is arranged behind a transparent element (17) in turn arranged near a delivery nozzle (12) of said sanitary appliance (11).

7. Device as in any claim hereinbefore, **characterized in that** each source of light (13) is associated by means of rapid coupling means (20) to a relative delivery nozzle (12).

8. Device as in claim 3, **characterized in that** said LEDs (13) are white, colored or multi-colored.

9. Device as in claim 3, **characterized in that** said LEDs (13) are commanded together according to the RGB technique in order to emit varying colored light.
